# FASCICULE DE BREVET EUROPEEN

(11) **EP 2 616 534 B1**
(45) Date de publication et mention de la délivrance du brevet: **20.01.2016**
(21) Numéro de dépôt: 11757846.8
(22) Date de dépôt: 13.09.2011
(51) Int. Cl.: C12M 1/02, C12M 1/34, C12M 1/00

(54) **DISPOSITIF DE CONTROLE DE LA TEMPERATURE D'UN PHOTOBIOREACTEUR SOLAIRE A ECLAIRAGE DIRECT**
VORRICHTUNG ZUR TEMPERATURREGELUNG EINES SONNENPHOTOBIOREAKTORS MIT DIREKTBELEUCHTUNG
DEVICE FOR CONTROLLING THE TEMPERATURE OF A DIRECT-ILLUMINATION SOLAR PHOTOBIOREACTOR

(30) Priorité: 13.09.2010 FR 1057285
(43) Date de publication de la demande: 24.07.2013
(73) Titulaire: Université de Nantes, 44000 Nantes (FR); Centre National de la Recherche Scientifique (CNRS), 75016 Paris (FR)
(72) Inventeur: GOETZ, Vincent, F-66450 Pollestres (FR); PRUVOST, Jérémy, F-44250 Saint-brevin Les Pins (FR); LEGRAND, Jack, F-44600 Saint Nazaire (FR); PLANTARD, Gael, F-11100 Narbonne (FR)
(74) Mandataire: Regimbeau
(86) Numéro de dépôt international: PCT/EP2011/065874
(87) Numéro de publication internationale: WO 2012/035027

(56) Documents cités:
- WO-A1-98/18903
- WO-A1-2010/011320
- NL-A- 9 100 715
- US-A- 2 646 972
- US-A1- 2009 023 199
- US-A1- 2009 155 864
- US-A1- 2009 291 490

## Description

### Domaine de l'invention

L'invention concerne le domaine des photo-réacteurs. Plus particulièrement, l'invention concerne le domaine des photo-réacteurs comprenant une chambre confinée de réaction. Elle s'applique notamment aux photo-bioréacteurs à écoulement pour l'écoulement en boucle fermée d'un liquide. Elle n'est cependant pas limitée à cette application précise et englobe également par exemple les réacteurs en cellules immobilisées, sans boucle de circulation, et les réacteurs en boucle ouverte.

### Arrière plan technologique

La production de biomasse par culture de microorganismes photosynthétiques via l'utilisation directe de l'énergie solaire s'inscrit pleinement dans le cadre du développement durable. Cette production est possible grâce aux photo-bioréacteurs à captation directe de lumière solaire, dans lesquels, la lumière du soleil est captée par une surface de captation et restituée aux microorganismes qui consomment une partie de ce rayonnement solaire pour leur photosynthèse. L'utilisation de photo-bioréacteur clos comprenant une chambre confinée de réaction, en opposition aux réacteurs en bassin ouvert, permet d'optimiser la production grâce à la possibilité de contrôler les conditions de croissance des microorganismes (notamment apport en divers gaz et nutriments).

Cependant, les photo-bioréacteurs à captation directe de lumière solaire clos sont susceptibles de subir un échauffement excessif de la culture de microorganisme. Cela est d'autant plus vrai que le volume de culture par surface de captation est faible dans ce type d'installation (par exemple, mais non limitativement de l'ordre de quelques litres par mètre carré de surface éclairée). Également, les microorganismes subissent les variations d'ensoleillement (cycle nycthéméral et annuel). Or la maîtrise de la température constitue un point clé pour le bon fonctionnement des photo-bioréacteurs. Cette température doit être contrôlée afin qu'elle se situe idéalement autour de l'optimum de croissance du microorganisme cultivé (usuellement située entre 25°C et 40°C). Si la température est trop importante, cela peut causer la mort des microorganismes.

Il existe des solutions qui concernent principalement le problème de la surchauffe du photo-bioréacteur à captation directe de lumière solaire clos.

Une solution consiste à asperger régulièrement d'eau le photo-bioréacteur. Une autre solution consiste à immerger partiellement au moins le photo-bioréacteur dans un bassin d'eau.

L'une comme l'autre de ces solutions a pour inconvénient de consommer beaucoup d'eau en raison du phénomène d'évaporation et de nécessiter la construction de bassins.

En outre, l'aspersion d'eau sur le photo-bioréacteur provoque l'encrassement des surfaces de captation de la lumière par dépôt de sels minéraux sur ces surfaces. Le flux lumineux arrivant dans la culture est donc diminué.

L'immersion en bassin provoque des problèmes de réflexion-absorption d'une partie du flux lumineux, diminuant également l'efficacité de captation du photo-bioréacteur.

Le document FR-A-2 914 315 décrit ainsi une installation pour la photosynthèse de microorganismes d'algue comprenant un dispositif de pulvérisation d'eau sur des canalisations, pour réduire la température du liquide de culture.

Le document US-2008/0160591 décrit un photo-bioréacteur placé dans un bassin d'eau à des fins de régulation thermique.

Le document WO-2008/008262 décrit un photo-bioréacteur comprenant un ensemble de transfert thermique à base de moyens de pulvérisation d'eau ou d'une fontaine.

D'autres solutions impliquent l'apport d'énergie électrique pour le refroidissement et/ou réchauffement actif(s) de la culture. Or, dans le cas d'une culture de microorganismes pour la production d'énergie, il est primordial et essentiel de minimiser tous les coûts énergétiques liés à la production des micro-organismes.

On trouvera des exemples de telles solutions dans les documents WO -2007/129327(qui divulgue un système de régulation thermique mettant en oeuvre un échangeur externe), US-2008/0220515 (qui prévoit un échange thermique avec un dispositif de régulation externe), FR-2 823 761 (qui propose un photo-bioréacteur comprenant une double enveloppe translucide externe permettant la circulation de fluides de thermorégulation), EP-1928994 (qui préconise une régulation thermique mettant en oeuvre des barrières thermiques associées aux tubes du réacteur, à base de sable, SiO2, verre, plastique ou céramique translucide), EP-0647707 (qui décrit un photo-bioréacteur comportant des parois thermiquement conductrices propres à être chauffées ou refroidies directement), US-4233958 (qui décrit un dôme reposant sur une semelle formant accumulateur de chaleur), US-2009/0291490 et WO 98/18903 (qui décrivent des photo-bioréacteurs avec une régulation thermique passive).

### Présentation de l'invention

Un des objectifs de l'invention est de palier au moins un des inconvénients de l'art antérieur décrit ci-dessus.

Pour cela, l'invention propose un photo-réacteur comprenant une chambre confinée de réaction, la chambre étant séparée de l'extérieur par une paroi de captation de lumière et une autre paroi, la paroi de captation et l'autre paroi étant parallèles entre elles ;
caractérisé en ce que le photo-réacteur comprend en plus une soupape thermique disposée contre l'autre paroi pour contrôler, de manière passive, l'augmentation de chaleur à l'intérieur de la chambre due au rayonnement traversant la paroi de captation afin de maintenir la température dans au moins une partie de la chambre sous une température seuil, la soupape thermique étant composé d'un matériau à changement de phase et un échangeur thermique selon la revendication 1.

Un avantage de ce photo-réacteur à régulation thermique passive réside en ce qu'il ne nécessite ni d'apport d'énergie ni d'eau pour permettre une régulation passive de la température au sein de la culture de microorganismes.

D'autres caractéristiques optionnelles et non limitatives sont les suivantes :
- le réacteur est un photo-bioréacteur ;
- le matériau est une paraffine ; laquelle a de préférence une plage de température de changement de phase entre 25°C et 40°C.

### Présentation des dessins

D'autres objectifs, caractéristiques et avantages de la présente invention apparaîtront à la lecture de la description détaillée qui suit, en référence aux dessins donnés à titre illustratif et non limitatif, parmi lesquels :
- la figure 1 est un premier exemple de réalisation d'un photo-bioréacteur;
- la figure 2 est un deuxième exemple de réalisation d'un photo-bioréacteur;
- la figure 3 est un troisième exemple de réalisation d'un photo-bioréacteur;
- la figure 4 est un quatrième exemple de réalisation d'un photo-bioréacteur; et
- les figures 5a à 5d montrent quatre diagrammes illustrant les courbes de température journalière au sein du photo-bioréacteur de la figure 1 en fonction de la masse de matériau à changement de phase utilisée pour la soupape thermique ;
- les figures 6a à 6c montrent trois diagrammes illustrant les courbes de température journalière au sein du photo-bioréacteur de la figure 1 en fonction des conditions d'échange en face arrière ;
- la figure 7a montre une courbe illustrant l'évolution de la densité de puissance d'irradiation lors d'une journée moyenne du mois de juillet à Nantes prise comme journée standard pour les diagrammes des figures 5a à 5d et 6a à 6c ;
- la figure 7b montre une courbe illustrant l'évolution de la température lors d'une journée moyenne du mois de juillet à Nantes prise comme journée standard pour les diagrammes des figures 5a à 5d et 6a à 6c ;
- la figure 8 représente schématiquement une vue en coupe verticale d'une variante de réalisation d'un photo-bioréacteur conforme à la présente invention, comportant un échangeur thermique ; et
- la figure 9 représente un exemple de courbe de transmission d'un verre filtrant le rayonnement infrarouge, susceptible d'être utilisé dans le cadre de la présente invention.

### Description détaillée

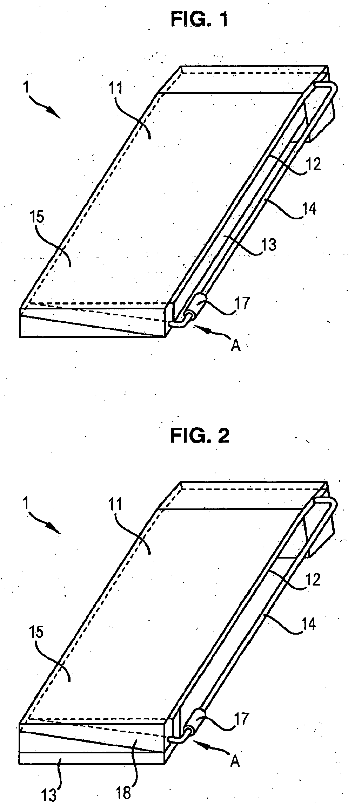

En référence aux figures 1 à 4, est ci-après décrit un exemple de photo-bioréacteur. Le photo-bioréacteur 1 permet la culture d'un ou de plusieurs types/espèces de microorganismes. Le terme « microorganismes » sera utilisé par la suite au pluriel mais englobe également le singulier.

Le photo-bioréacteur 1 comprend une chambre 15 confinée de réaction, ici une chambre à écoulement pour l'écoulement en boucle fermée d'un liquide.

La chambre 15 de réaction est comprise entre deux parois :
- une paroi 11 de captation de lumière la séparant de l'extérieur, par laquelle traverse le rayonnement solaire ; et
- une autre paroi 12 qui peut être parallèle à la paroi 11 de captation.

La distance entre la paroi 11 de captation et l'autre paroi 12 est choisie de manière à permettre un écoulement satisfaisant dans la chambre 15 de réaction, entre ces deux parois 11 et 12.

L'écoulement en boucle fermée est assuré par un mécanisme 14 de remontée de liquide, lequel peut faire l'objet de nombreux modes de réalisation bien connus de l'homme du métier. Par exemple, le mécanisme 14 de remontée comprend une rampe de remontée de fluide dont une extrémité est située en aval de l'écoulement de la culture, en partie inférieure de la chambre 15, et l'autre extrémité est située en amont, en partie supérieure de la chambre 15. Le mécanisme 14 de remontée comprend également une pompe pour faire circuler le liquide vers l'amont de la chambre 15 de réaction. La pompe occasionne un écoulement le long de la rampe de remontée dans un sens contraire à l'écoulement de la culture. Un tel réacteur est décrit dans la demande de brevet français n° FR09 568 70.

Le photo-bioréacteur 1 comprend en plus, selon l'invention, une soupape 13 thermique pour maintenir, de manière passive, la température sous une température seuil Ts dans au moins une partie de la chambre 15 de réaction. La soupape 13 thermique peut être posée contre l'autre paroi 12, en la positionnant soit à l'intérieur de la chambre 15 de réaction, soit à l'extérieur.

La température seuil Ts est déterminée par les microorganismes présents dans la culture. Ainsi, la température seuil Ts est choisie de manière à être inférieure à la température maximale que peut supporter l'ensemble des microorganismes cultivés. La température seuil Ts peut être supérieure à la température maximale que peut supporter un microorganisme non désiré au sein de la culture.

Dans le cadre de la présente invention, la soupape thermique 13 est formée à base d'un matériau à changement de phase organique ou inorganique dont la température de changement de phase est adaptée à la température seuil Ts souhaitée.

Le matériau composant la soupape thermique 13 peut être formé par exemple de paraffine.

A titre d'exemple non limitatif et dans le cas d'une température seuil Ts de 30°C, un matériau particulièrement bien adapté et disponible commercialement est constitué de la paraffine RT31 (Rubitherm) qui présente une zone de fusion de 27 à 31 °C pour une enthalpie de fusion de 170 kJ/kg.

L'efficacité du transfert thermique entre la culture et l'autre paroi 12 dépend des conditions d'écoulement. Celle entre l'autre paroi 12 et la soupape 13 thermique dépend d'un coefficient d'échange entre le matériau de l'autre paroi 12 et celui de la soupape 13 thermique et de la conductivité thermique de la soupape 13 thermique.

Dans le cadre d'une soupape 13 thermique en matériau à changement de phase, l'efficacité du transfert thermique entre la soupape 13 thermique et l'autre paroi 12 est améliorée si le matériau à changement de phase est au sein d'une matrice en graphite.

Pendant toute la durée du changement de phase, la température du matériau à changement de phase est sensiblement constante. C'est-à-dire que si l'on doit chauffer pour atteindre la température de changement de phase, qui est de préférence comprise entre 25°C et 40°C, la température du matériau, qui ne subit pas encore un changement de phase, va progressivement augmenter jusqu'à atteindre la température de changement de phase. A cette température, le matériau à changement de phase va passer d'un premier état à un deuxième état. Tant qu'il reste encore du matériau dans le premier état, la température reste à la température de changement de phase. La hausse de la température du matériau ne recommencera qu'une fois que le matériau se trouvera entièrement dans le deuxième état.

Pour la composition du matériau à changement de phase formant la soupape thermique 13, dans la gamme de température 30°C, on peut citer les produits suivants :
- alcanes ou paraffines : n-octadécane, nonadécane, produits commercialisés sous la dénomination RT42, RT31 ou RT27 (mélanges de paraffine, produits Rubitherm);
- matériaux organiques autres que paraffine : acide caprique (CH₃(CH₂)₈COOH), 1-dodécanol (CH₃(CH₂)₁₁OH), thioglycolate d'octadécyle, palmitate de méthyle, stéarate de méthyle, stéarate d'éthyle (et mélange de ces trois derniers constituants), acide lactique, stéarate de vinyle ;
- matériaux inorganiques : chlorure de calcium hexahydraté (CaCl₂·6H₂O), nitrate de manganèse hexahydraté (Mn(NO₃)₂·6H₂O), nitrate de lithium trihydraté (LiNO₃·3H₂O)**,** sulfate de sodium décahydraté (Na₂SO₄·10H₂O) ;
- eutectiques inorganiques: chlorure de calcium avec chlorure de magnésium hexahydraté ; nitrate de calcium tétrahydraté avec nitrate de zinc hexahydraté ; chlorure de calcium, de sodium et de potassium avec de l'eau ; sulfate de sodium décahydraté avec de l'eau.

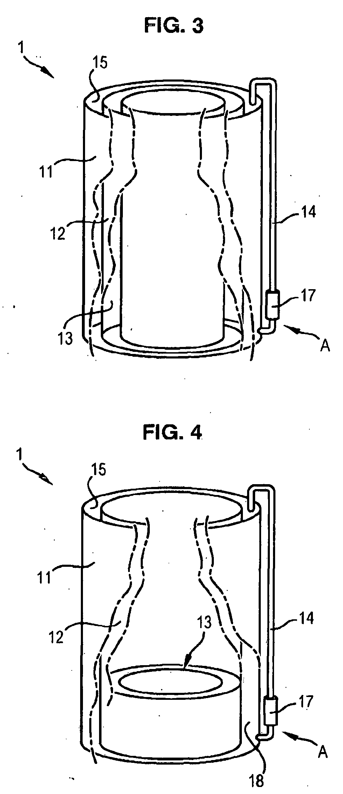

La soupape 13 thermique en matériau à changement de phase peut recouvrir la totalité de l'autre paroi 12. Ainsi la température de la culture est maintenue sous la température seuil Ts dans toute la chambre 15 de réaction (voir les figures 1 et 3).

La soupape 13 thermique en matériau à changement de phase peut ne recouvrir qu'une partie de l'autre paroi 12, voire être au contact d'une partie du photo-bioréacteur 1 qui ne soit pas dans la chambre 15 de réaction mais dans une chambre 18 de sécurité en aval de l'écoulement du liquide que constitue la culture de microorganismes.

Sur les figures 2 et 4, la chambre de sécurité 18 correspond à un compartiment situé en partie basse du photo-bioréacteur 1 et dans lequel est susceptible de s'accumuler le liquide contenu dans la chambre de réaction 15, en cas d'interruption de l'écoulement.

Le photo-bioréacteur 1 peut comprendre également un régulateur 17 d'écoulement pour couper le mécanisme 14 de remontée et ainsi la boucle d'écoulement du liquide à l'intérieur de la chambre 15 de réaction lorsque la température dans la chambre 15 de réaction dépasse une autre température seuil Ts' inférieure ou égale à la température seuil Ts. Le liquide s'accumule alors en aval de l'écoulement, éventuellement dans la chambre 18 de sécurité si celle-ci est prévue. Ainsi, quand la température dans la chambre 15 de réaction dépasse l'autre température seuil Ts', le liquide est confiné dans un espace de la chambre 15 de réaction ou dans la chambre 18 de sécurité là où est présente la soupape 13 thermique (voir les figures 2 et 4).

Le matériau à changement de phase sert également de stockage d'énergie. En effet en chauffant, puis en changeant d'état, le matériau à changement de phase emmagasine l'énergie solaire (celle qui est due au rayonnement non consommé par la photosynthèse) et la restitue quand le rayonnement du soleil devient insuffisant (par exemple en fin de journée) garantissant le maintien de la température optimale de croissance des microorganismes plus longtemps.

Le photo-bioréacteur 1 peut être un photo-bioréacteur plan, comme illustré dans les figures 1 et 2. Dans ce cas, la paroi supérieure 11 de captation et l'autre paroi inférieure 12 sont planes, parallèles entre elles et inclinées par rapport au sol assurant ainsi un écoulement par gravité. La paroi 11 de captation est alors disposée au-dessus de l'autre paroi 12, cette disposition étant imposée par la géométrie pour pouvoir capter directement la lumière solaire. En partie inférieure, le fond de la chambre de sécurité 18 comprend par ailleurs un pan incliné vers le bas en direction du point d'entrée du conduit de remontée 14.

La face avant de captation 11 est formée typiquement d'un vitrage de quelques mm d'épaisseur.

La face arrière 12 est formée d'un panneau en matériau approprié, par exemple en métal, verre ou polymère.

La soupape 13 thermique peut couvrir entièrement l'autre paroi 12 soit par le dessus (auquel cas, la soupape 13 thermique est à l'intérieur de la chambre 15 de réaction), soit par le dessous (voir figure 1). L'autre paroi 12 et la soupape 13 thermique sont en contact l'une de l'autre pour permettre un transfert thermique.

La soupape 13 thermique peut ne couvrir qu'une chambre 18 de sécurité prévue dans le photo-bioréacteur 1, soit par le dessus, soit par le dessous (voir figure 2). La soupape 13 thermique est positionnée en contact avec une paroi de la chambre 18 pour assurer le transfert thermique.

Le photo-bioréacteur 1 peut aussi être un photo-bioréacteur cylindrique, comme illustré dans les figures 3 et 4. Dans ce cas, la paroi 11 de captation et l'autre paroi 12 ont une géométrie cylindrique et centrée sur le même axe, de préférence vertical. La paroi 11 de captation est à l'extérieur alors que l'autre paroi 12 est à l'intérieur, cette disposition étant imposée par la géométrie pour pouvoir capter directement la lumière solaire. L'écoulement du liquide formé par la culture de microorganisme se fait de haut en bas par gravité.

La soupape 13 thermique peut couvrir entièrement l'autre paroi 12 soit par l'extérieur du cylindre formé par l'autre paroi 12 (auquel cas, la soupape 13 thermique est à l'intérieur de la chambre 15 de réaction), soit par l'intérieur du cylindre formé par l'autre paroi 12 (auquel cas, la soupape 13 thermique est à l'extérieur de la chambre 15 de réaction, voir figure 3). L'autre paroi 12 et la soupape 13 thermique sont en contact l'une de l'autre pour permettre un transfert thermique.

La soupape 13 thermique peut ne couvrir qu'une partie de l'autre paroi 12 soit par l'extérieur (voir figure 4), soit par l'intérieur, du cylindre formé par l'autre paroi 12. La soupape 13 thermique est positionnée en contact avec la partie basse de l'autre paroi 12 pour assurer le transfert thermique et la fonction de sécurité dans cette partie de sécurité. En effet, lorsque le mécanisme 14 de remontée est coupé, le liquide que constitue la culture s'écoule vers le bas dans la partie de sécurité 18.

Le photo-bioréacteur 1 peut comprendre en outre un filtre proche infrarouge et ou ultraviolet sur ou sous la paroi 11 de captation de lumière. Le filtre est transparent aux longueurs d'onde du domaine visible. Prévoir un filtre est avantageux en ce que tout le rayonnement solaire n'est pas utile. En effet, seule la partie du rayonnement solaire correspondant aux longueurs d'onde situées dans le domaine du visible est utile aux microorganismes photosynthétiques avec un rendement maximum de 15%. Une grande partie du rayonnement solaire entrant dans le réacteur a donc pour conséquence de chauffer la chambre 15 de réaction.

L'efficacité du transfert thermique entre la culture et l'autre paroi 12 dépend des conditions d'écoulement. Celle entre l'autre paroi 12 et la soupape 13 thermique dépend d'un coefficient d'échange entre le matériau de l'autre paroi 12 et celui de la soupape 13 thermique et de la conductivité thermique de la soupape 13 thermique.

Dans le cadre d'une soupape 13 thermique en matériau à changement de phase, l'efficacité du transfert thermique entre la soupape 13 thermique et l'autre paroi 12 est améliorée si le matériau à changement de phase est au sein d'une matrice en graphite.

Le reste du rayonnement peut être néfaste (ultraviolets représentant 5% de la densité de puissance totale du spectre solaire standard, soit environ 50 W·m⁻²) ou peut causer la surchauffe du photo-bioréacteur 1 (proche infrarouge représentant 52% de la densité de puissance totale du spectre solaire standard, soit environ 515 W·m⁻²). En outre, comme la capacité de la soupape 13 thermique en matériau à changement de phase à maintenir la chambre 15 de culture sous la température seuil Ts dépend de sa masse, prévoir un filtre permet de réduire la masse nécessaire, puisqu'une partie des rayonnements chauffant n'entre pas dans la chambre 15 de réaction.

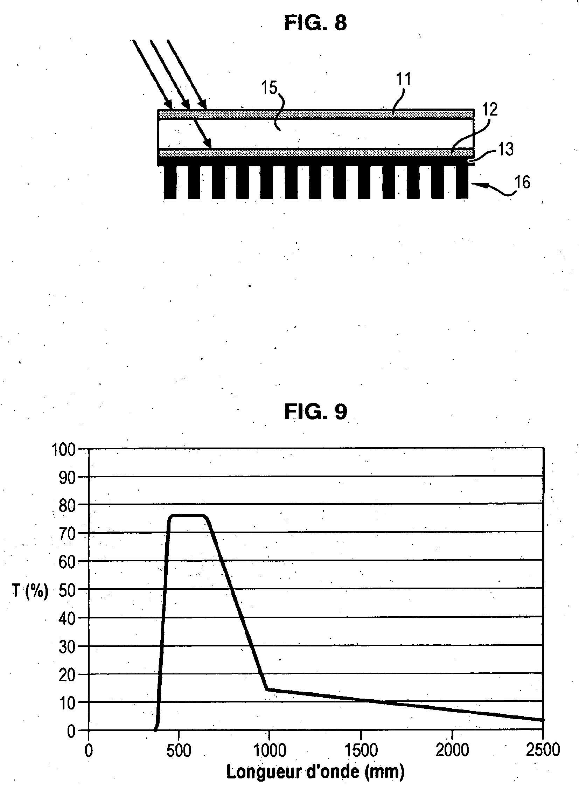

La paroi 11 de captation peut jouer le rôle de filtre envers le rayonnement qui n'est pas utile. Par exemple, une paroi 11 de captation en verre classique joue naturellement le rôle de filtre pour le rayonnement ultraviolet compris dans le spectre solaire. Des verres techniques assurent la fonction de filtre du rayonnement proche infrarouge (longueurs d'onde comprises entre 700 nm et 3000 nm). On a représenté sur la figure 9 annexée le pourcentage de transmission de verres disponibles dans le commerce destinés à la filtration du rayonnement infrarouge et proche infrarouge. D'autres matériaux peuvent être utilisés afin de se rapprocher idéalement d'une transparence parfaite aux longueurs d'onde utiles à la photosynthèse situées dans le domaine visible et une réflexion totale aux longueurs d'onde ultraviolet et surtout proche infrarouge.

Comme on l'a représenté schématiquement sur la figure 8, le photo-réacteur 1 comprend un échangeur 16 thermique. L'échangeur thermique 16 est disposé en contact avec la soupape 13 thermique lorsque celle-ci est prévue à l'extérieur de la chambre de réaction 15. L'échangeur thermique 16 peut également être disposé en contact avec l'autre paroi 12, notamment lorsque la soupape 13 thermique est prévue à l'intérieur de la chambre de réaction 15. L'échangeur thermique 16 permet de soulager la soupape 13 thermique en assurant un certain refroidissement de celle-ci.

Selon d'autres variantes non revendiquées, l'échangeur 16 thermique peut également avoir une autre position, tant qu'il puisse assurer un transfert thermique entre la chambre 15 de réaction et l'extérieur à travers éventuellement un élément du photo-bioréacteur 1.

L'échangeur 16 thermique peut être un radiateur à ailettes.

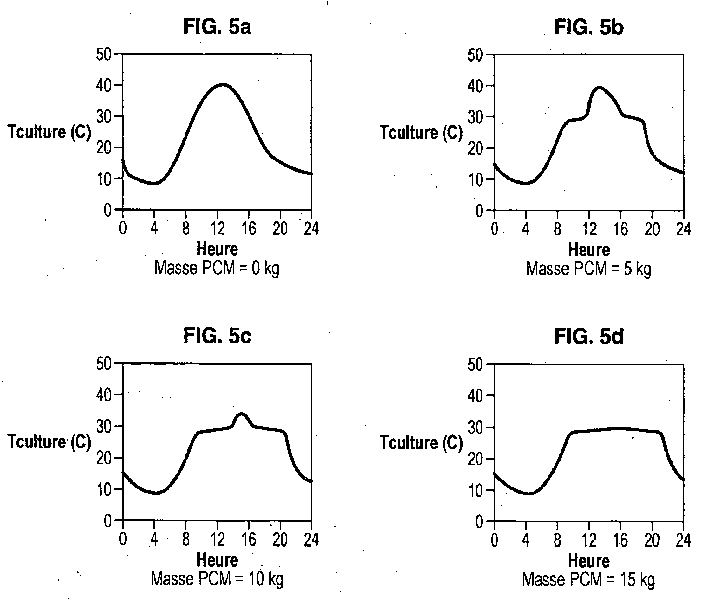

Les figures 5a à 5d illustrent les résultats d'un calcul théorique du réchauffement de la culture au sein de la chambre 15 de réaction pendant une journée standard lorsque le photo-bioréacteur 1 ne comprend pas de soupape 13 thermique (figure 5a) ou comprend une soupape 13 thermique en matériau à changement de phase ayant une épaisseur de 1 cm (figure 5b), 2 cm (figure 5c) et 3 cm (figure 5d).

Les calculs conduisant aux figures 5a à 5d ont été effectués pour un photo-bioréacteur 1 comprenant un filtre en verre présentant une transmission et une réflexion du rayonnement solaire de 0,5. Les conditions d'échanges avec le milieu ambiant, des surfaces 11 et 12 (Figure 5a) ou de la surface de la soupape 13 thermique (Figure 5b, 5c, 5d), retenues pour le calcul, correspondent à un coefficient d'échange par convection naturelle de 5 W·m⁻²·K⁻¹. La soupape thermique utilisée dans la simulation comporte une surface de 0,7 m², et est en matériau à changement de phase formé de Rubitherm ayant respectivement des épaisseurs de 1 cm, 2 cm et 3 cm correspondant à des masses de 5 kg, 10 kg et 15 kg.

On voit sur la figure 5a que lorsque le photo-bioréacteur 1 ne comprend pas de soupape 13 thermique (figure 5a), la température au sein de la chambre 15 de réaction atteint un maximum de 40°C entre 12:00 et 13:00 et reste supérieure à 30°C pendant près de sept heures.

Lorsque le photo-bioréacteur 1 comprend une soupape 13 thermique en matériau à changement de phase de 1 cm (figure 5b), la température au sein de la chambre 15 de réaction atteint également un maximum de 40°C aux alentours de 13:00 et reste supérieure à 30°C pendant quatre heures, soit trois heures de moins que dans le cas illustré sur la figure 5a.

Lorsque la soupape 13 thermique est de 2 cm (figure 5c), la température au sein de la chambre 15 de réaction reste en dessous de 35°C le long de la journée et supérieure à 30°C pendant deux heures et vingt minutes environ, soit près de quatre heures et quarante minutes de moins que dans le cas illustré sur la figure 5a. Le pic de température est déplacé aux alentours de 14:45.

Enfin, lorsque la soupape 13 thermique est de 3 cm, la température au sein de la chambre 15 de réaction reste constamment en dessous de 30°C (figure 5d).

Ces comparaisons démontrent l'efficacité de la soupape 13 thermique en matériau à changement de phase.

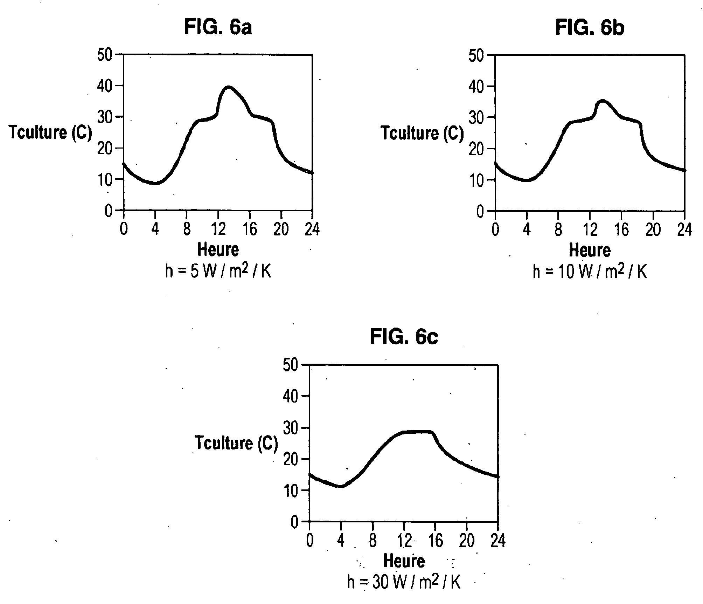

Les figures 6a à 6c illustrent le réchauffement de la culture au sein de la chambre 15 de réaction pendant une journée standard lorsque le photo-bioréacteur 1 comprend un filtre en verre, une soupape 13 thermique en matériau à changement de phase ayant une épaisseur de 1 cm sans échangeur thermique (figure 6a) : et avec un échangeur thermique développant une surface ailettée (comprenant des ailettes) permettant d'augmenter la surface d'échange initiale (ici 0,7m²) d'un facteur 2 (figure 6b) ou d'un facteur 6 (figure 6c). Les calculs présentés ici à titre illustratif ont été effectués dans le cas d'un échange dans des conditions de convection naturelle conduisant à un coefficient d'échange de 5 W·m⁻²·K⁻¹,

La figure 6a correspond à la figure 5b. Elle ne sera donc pas davantage commentée.

Lorsque l'échangeur thermique en face arrière permet de doubler la surface d'échange (figure 6b), la température au sein de la chambre 15 de réaction ne dépasse pas 35°C pendant toute la journée standard et reste au dessus de 30°C pendant moins de trois heures et vingt minutes, rapprochant le résultat obtenu au cas illustré par la figure 5c.

Lorsque l'échangeur thermique en face arrière développe une surface six fois plus importante (figure 6c), la température au sein de la chambre 15 de réaction ne dépasse pas les 30°C pendant toute la journée standard. Le résultat est même amélioré par rapport au cas illustré par la figure 5d.

Ainsi, on peut voir que l'utilisation d'un échangeur thermique en face arrière améliore les résultats obtenus. Ceci permet de réduire la masse de matériau à changement de phase nécessaire.

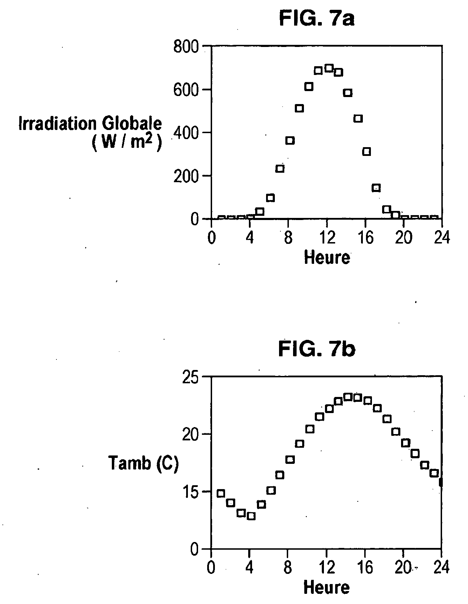

L'évolution de la température d'une journée standard telle qu'envisagée lors des simulations aboutissant aux résultats des figures 5a à 5d et 6a à 6c est illustrée sur la figure 7b. Cette évolution correspond à celle d'une journée moyenne du mois de juillet à Nantes. L'évolution au cours de la journée de la densité de flux correspondante au rayonnement solaire est illustrée sur la figure 7a.

La description a été faite ci-dessus par référence à un photo-bioréacteur, mais elle peut également être facilement adaptée pour tout type de réacteur à captation directe de lumière solaire, par exemple un photo-réacteur opérant dans le domaine de la photocatalyse pour le traitement de liquides. Également, les géométries peuvent varier et l'homme du métier saura adapter l'enseignement de la présente description à ces diverses géométries.

Les apports en gaz A, notamment CO2, et en nutriment se font par des conduits dédiés connus de l'homme du métier, par exemple au niveau du conduit 14. De même le soutirage pour la récolte des micro-organismes s'effectue par tous moyens appropriés connus de l'homme de l'art, par exemple à l'aide de moyens prévus à cet effet sur le conduit 14, lorsque les conditions sont réunies.

L'homme de l'art appréciera à la lecture de la description qui précède que la présente invention permet des avantages décisifs quant à la régulation de température, vis-à-vis de l'art antérieur, par mise en oeuvre d'un système de régulation passif comprenant une soupape thermique et un échangeur thermique.

Il n'est pas nécessaire de disposer une pompe sur le conduit 14 lorsque le liquide est mis en mouvement par d'autres moyens, par exemple comme cela est connu en soi, par la pression statique différentielle résultant de l'injection de gaz dans la chambre de réaction 15.

## Revendications

1. Photo-réacteur (1) comprenant une chambre (15) confinée de réaction, la chambre (15) étant séparée de l'extérieur par une paroi (11) de captation de lumière et une autre paroi (12), la paroi de captation et l'autre paroi étant parallèles entre elles ;
le photo-réacteur (1) comprend en plus une soupape (13) thermique disposée contre l'autre paroi (12) pour contrôler, de manière passive, l'augmentation de chaleur à l'intérieur de la chambre (15) due au rayonnement traversant la paroi (11) de captation afin de maintenir la température dans au moins une partie de la chambre (15) sous une température seuil (Ts), la soupape thermique (13) étant composé d'un matériau à changement de phase ;
**caractérisé en ce que** photo-réacteur (1) comprend un échangeur (16) thermique disposé contre la soupape (13) thermique, le cas échéant, à l'opposé de l'autre paroi et assurant un échange thermique entre la soupape (13) thermique et l'extérieur.

2. Photo-réacteur (1) selon la revendication 1, **caractérisé en ce que** le réacteur est un photo-bioréacteur.

3. Photo-réacteur (1) selon la revendication 1 ou 2, dans lequel le matériau est une paraffine.

4. Photo-réacteur (1) selon la revendication 3, dans lequel le matériau est une paraffine avec une plage de température de changement de phase proche de 30°C.

5. Photo-réacteur (1) selon la revendication 1 ou 2, dans lequel le matériau est choisi parmi les produits suivants :
- alcanes ou paraffines : notamment n-octadécane, nonadécane,
- matériaux organiques autres que paraffine : notamment acide caprique; 1-dodécanol, thioglycolate d'octadécyle ; palmitate de méthyle, stéarate de méthyle, stéarate d'éthyle, et mélange de ces trois derniers constituants ; acide lactique ; stéarate de vinyle ;
- matériaux inorganiques : notamment chlorure de calcium hexahydraté ; nitrate de manganèse hexahydraté ; nitrate de lithium trihydraté ; sulfate de sodium décahydraté ;
- eutectiques inorganiques : notamment chlorure de calcium avec chlorure de magnésium hexahydraté ; nitrate de calcium tétrahydraté avec nitrate de zinc hexahydraté ; chlorure de calcium, de sodium et de potassium avec de l'eau ; sulfate de sodium décahydraté avec de l'eau.

6. Photo-réacteur selon l'une des revendications 1 à 5, dans lequel la paroi (11) de captation et l'autre paroi (12) sont planes et inclinées par rapport au sol assurant ainsi un écoulement, la paroi (11) de captation étant disposée au-dessus de l'autre paroi (12).

7. Photo-réacteur (1) selon l'une des revendications 1 à 6, comprenant en outre un moyen formant filtre laissant passer uniquement les rayonnements utiles.

8. Photo-réacteur selon l'une des revendications 1 à .7, dans lequel l'échangeur (16) thermique est un radiateur à ailette.

9. Photo-réacteur selon l'une des revendications 1 à 8, dans lequel la soupape (13) thermique est disposée en aval de l'écoulement, et comprenant en outre un régulateur (17) d'écoulement pour couper la boucle (14) d'écoulement du liquide à l'intérieur de la chambre (15) de réaction lorsque la température dans la chambre (15) de réaction dépasse une autre température seuil (Ts'), le liquide s'accumulant en aval de l'écoulement.

## Patentansprüche

1. Photoreaktor (1), umfassend eine abgeschlossene Reaktionskammer (15), wobei die Kammer (15) vom Außenbereich durch eine Wand (11) zur Erfassung von Licht und eine andere Wand (12) getrennt ist, wobei die Wand zur Erfassung und die andere Wand untereinander parallel sind;
der Photoreaktor (1) umfasst außerdem ein thermisches Ventil (13), das gegen die andere Wand (12) angebracht ist, um auf passive Weise die Erhöhung der Wärme im Inneren der Kammer (15) aufgrund der Strahlung, die die Wand (11) zur Erfassung durchdringt, zu steuern, um die Temperatur in mindestens einem Teil der Kammer (15) unter einer Schwellentemperatur (Ts) zu halten, wobei das thermische Ventil (13) aus einem Phasenwechselmaterial zusammengesetzt ist,
**dadurch gekennzeichnet, dass** der Photoreaktor (1) einen Wärmetauscher (16) umfasst, der gegen das thermische Ventil (13) angebracht ist, gegebenenfalls gegenüber der anderen Wand, und einen Wärmetausch zwischen dem thermischen Ventil (13) und dem Außenbereich sicherstellt.

2. Photoreaktor (1) nach Anspruch 1, **dadurch gekennzeichnet, dass** der Reaktor ein Photobioreaktor ist.

3. Photoreaktor (1) nach Anspruch 1 oder 2, wobei das Material ein Paraffin ist.

4. Photoreaktor (1) nach Anspruch 3, wobei das Material ein Paraffin mit einem Temperaturbereich des Phasenwechsels nahe 30 °C ist.

5. Photoreaktor (1) nach Anspruch 1 oder 2, wobei das Material ausgewählt ist aus den folgenden Produkten:
- Alkanen oder Paraffinen: insbesondere n-Octadecan, Nonadecan,
- organischen Materialien außer Paraffin: insbesondere Caprinsäure; 1-Dodecanol, Octadecylthioglycolat; Methylpalmitat, Methylstearat, Ethylstearat, und einer Mischung dieser drei Bestandteile; Milchsäure; Vinylstearat;
- anorganischen Materialien, insbesondere hexahydriertem Calciumchlorid; hexahydriertem Mangannitrat; trihydriertem Lithiumnitrat; decahydriertem Natriumsulfat;
- anorganischem Eutektika; insbesondere Calciumchlorid mit hexahydriertem Magnesiumchlorid; tetrahydriertem Calciumnitrat mit hexahydriertem Zinknitrat; Calcium- Natrium- und Kaliumchlorid mit Wasser; decahydriertem Natriumsulfat mit Wasser.

6. Photoreaktor nach einem der Ansprüche 1 bis 5, wobei die Wand (11) zur Erfassung und die andere Wand (12) eben und geneigt mit Bezug auf den Boden sind, wodurch ein Abfluss sichergestellt wird, wobei die Wand (11) zur Erfassung über der anderen Wand (12) angeordnet ist.

7. Photoreaktor (1) nach einem der Ansprüche 1 bis 6, außerdem umfassend ein Mittel, das einen Filter bildet, der nur die nützlichen Strahlen passieren lässt.

8. Photoreaktor nach einem der Ansprüche 1 bis 7, wobei der Wärmetauscher (16) ein Rippenkühler ist.

9. Photoreaktor nach einem der Ansprüche 1 bis 8, wobei das thermische Ventil (13) nachgelagert vom Abfluss angeordnet ist und außerdem einen Abflussregler (17) umfasst, um die Schleife (14) des Abflusses der Flüssigkeit im Inneren der Reaktionskammer (15) zu trennen, wenn die Temperatur in der Reaktionskammer (15) eine andere Temperaturschwelle (Ts') übersteigt, wobei sich die Flüssigkeit nachgeordnet vom Abfluss ansammelt.

## Claims

1. Photoreactor (1) comprising a contained reaction chamber (15), wherein the chamber (15) is separated from the exterior by a light-capturing wall (11) and another wall (12), the capturing wall and the other wall being parallel to each other;
the photoreactor (1) additionally comprises a thermal valve (13) placed against the other wall (12) for passively controlling the increase in heat inside the chamber (15) due to the radiation passing through the capturing wall (11) for maintaining the temperature in at least one part of the chamber (15) under a threshold temperature (Ts), the thermal valve (13) being made of a phase-change material;
**characterised in that** the photoreactor (1) comprises a heat exchanger (16) placed against the thermal valve (13), if applicable, opposite the other wall and ensuring heat exchange between the thermal valve (13) and the exterior.

2. Photoreactor (1) according to claim 1, **characterised in that** the reactor is a photobioreactor.

3. Photoreactor (1) according to claim 1 or 2, **characterised in that** the material is a paraffin.

4. Photoreactor (1) according to claim 3, **characterised in that** the material is a paraffin with a phase-change temperature range close to 30°C.

5. Photoreactor (1) according to claim 1 or 2, **characterised in that** the material is chosen from the following products:
- alkanes or paraffins: in particular n-octadecane, nonadecane;
- organic materials other than paraffin: in particular capric acid; 1-dodecanol, octadecyl thioglycolate; methyl palmitate, methyl stearate, ethyl stearate, and a mixture of the last three components; lactic acid; vinyl stearate;
- inorganic materials: in particular calcium chloride hexahydrate; manganese nitrate hexahydrate; lithium nitrate trihydrate; sodium sulphate decahydrate;
- inorganic eutectics: in particular calcium chloride with magnesium chloride hexahydrate; calcium nitrate tetrahydrate with zinc nitrate hexahydrate; calcium chloride, sodium chloride and potassium chloride with water; sodium sulphate decahydrate with water.

6. Photoreactor according to one of claims 1 to 5, wherein the capturing wall (11) and the other wall (12) are flat and sloping in relation to the ground thereby ensuring a flow, the capturing wall (11) being placed above the other wall (12).

7. Photoreactor (1) according to one of claims 1 to 6, further comprising a filter-forming means only allowing useful radiation to pass through.

8. Photoreactor (1) according to one of claims 1 to 7, wherein the heat exchanger (16) is a finned radiator.

9. Photoreactor according to one of claims 1 to 8, wherein the thermal valve (13) is placed downstream of the flow, and further comprising a flow regulator (17) to cut the flow loop (14) of the liquid inside the reaction chamber (15) when the temperature in the reaction chamber (15) exceeds another threshold temperature (Ts'), the liquid accumulating downstream of the flow.
